# EUROPEAN PATENT APPLICATION

(11) **EP 1 352 670 A1**
(43) Date of publication of application: **15.10.2003**
(21) Application number: 03076089.6
(22) Date of filing: 11.04.2003
(51) Int. Cl.: A61M 16/10

(54) **Coaxial breathing system**

(30) Priority: 11.04.2002 GB 0208358
(71) Applicant: Armstrong Medical Limited, Coleraine, County Londonderry BT52 1BS (GB)
(72) Inventor: Magee, Ciaran, Coleraine, Norther Ireland BT51 3PH (GB); Armstrong, John, Coleraine, Northern Ireland BT53 7PU (GB)
(74) Representative: O'Connell, Maura, Dr.

(57) **Abstract**

The invention provides a coaxial breathing system having system user distal (16a,b) and proximal ends (18). The coaxial breathing system comprises an inlet tube (12) for conveying inspiratory gases and an outlet tube (14) for conveying expiratory gases, each of the inlet and outlet tubes having tube user distal and proximal ends, the tube user distal ends being in fluid communication with, in use, a fluid supply means (33) and the tube user proximal ends being in fluid communication with, in use, a user, the inlet tube being coaxially arranged within the outlet tube; a temperature sensor (64), in use, at the system user proximal end; a heat source consisting essentially of a heater wire (28) surrounded by an insulating hydrophobic layer and located in the outlet tube, the heat source being dimensioned and arranged to heat at least the expiratory gases in the outlet tube; and a regulating means for controlling actuation of the heat source, the regulating means being in operative association with the temperature sensor.

The coaxial breathing system of the present invention has utility in optimising the temperature and humidity of gas as inhaled by the patient.

## Description

The present invention relates to a coaxial breathing system, in which an inlet tube is located within an outlet tube.

Coaxial breathing systems remove the requirement to tape or otherwise support two separate inlet and outlet tubes and, thereby minimise entanglements. In addition, a coaxial system, in which the inlet tube is housed within the outlet tube, reduces, to some extent, the problem of gases cooling during travel down the inlet tube prior to entry into the patient. In such a coaxial system there is, in effect, a counter-current gas flow of the inspiratory, versus the expiratory, gases. The entire coaxial breathing system forms a heat exchanger such that the warmer expiratory gases flow counter-currently with respect to the cooler inspiratory gases and tend to warm the inspiratory gases during counter-current movement. Thus, the inspiratory gases are warmed throughout the entire path of travel of the gases, from the gas supply system to the patient, since heat is exchanged across the inner conduit wall from the warmer expiratory gases to the cooler inspiratory gases.

EP-A-1 166 814 concerns itself with such a coaxial breathing system, in which an inlet tube is located within an outlet tube. More specifically, EP-A-1 166 814 discloses, at column 1, lines 40-41, the inclusion of a heating means located within the expiratory gas flow path. The heating means is in the form of a "heated wick". This is defined at paragraph 8 as being a heater associated with a portion of hydrophilic material. EP-A-1 166 814 relies on the presence of water condensate in the outlet tube - without this condensate, there could not be observed a "wicking" effect. In contrast, the present invention requires a heat source comprising a heater wire consisting essentially of a heater wire covered by an insulating layer, i.e., there is no hydrophilic material around the insulating layer.

DE-A-37 30 551 describes a coaxial system, in which the inlet tube is housed within the outlet tube. Figures 1 and 2 of DE-A-37 30 551 suggest that a heating element, in the form of a wire coil, surrounds the outer part of the entire coaxial system.

US-A-4,967,744 discloses a coaxial system, in which the inlet tube is housed within the outlet tube. More specifically, column 2, lines 60-68 discloses the presence of a heating wire 42, which is wrapped around the inlet tube 40. Claims 2 and 12 recite that the heating wire is wound about the inner tube wall and Claims 13, 14 and 15 each recite that the heating means is disposed between the outer tube and the inner tube.

None of these prior art documents identifies the power density required in the outlet tube, or in both the inlet and outlet tubes, to achieve a desired temperature and / or humidity in an inhaled breath.

None of these prior art documents identifies that the location of the temperature sensor plays a role in optimising temperature and / or humidity in an inhaled breath.

It is an object of the present invention to alleviate some or all of the problems associated with the prior art.

It is a further object of the present invention to modify a coaxial breathing system, in which an inlet tube is located within an outlet tube, to facilitate the conveying of a gas, or a mixture thereof, along an inlet tube at a temperature above 15°C, preferably above 25°C, more preferably above 30°C, even more preferably above 35°C, most preferably above 36.5°C. The gas, or the mixture thereof, being conveyed along the inlet tube at an elevated temperature has, preferably, a relative humidity of greater than 30%, preferably greater than 50%, more preferably greater than 75%, most preferably greater than 85%.

It is a further object of the present invention to modify a coaxial breathing system, in which an inlet tube is located within an outlet tube, to facilitate the conveying of a gas, or a mixture thereof, along the outlet tube at a temperature above 15°C, preferably above 25°C, more preferably above 30°C, even more preferably above 35°C, most preferably above 36.5°C. The gas, or the mixture thereof, being conveyed along the outlet tube at an elevated temperature has, preferably, a relative humidity of greater than 30%, preferably greater than 50%, more preferably greater than 75%, most preferably greater than 85%.

It is an object of the present invention to devise a coaxial breathing system which is suitable for conveying inspiratory gases along an inlet tube of a coaxial breathing system at a temperature in the range of 36.5 - 39.5°C and at a relative humidity in the range of 100-95% to a user proximal end of the coaxial breathing system over a period of at least 2 hours, preferably at least 12 or 24 hours, more preferably at least 3 days. Suitably, the inspiratory gases enter the inlet tube of the coaxial breathing system at a temperature in the range of 33-37.5°C. Suitably, the inspiratory gases exit the coaxial breathing system at a temperature in the range of 37 - 40, preferably 37.5 - 39.5, °C.

It is a further object of the present invention to devise a coaxial breathing system which is suitable for conveying expiratory gases along an outlet tube of a coaxial breathing system at a temperature in the range of 33 - 39.5°C and at a relative humidity in the range of 100-95% to a user distal end of the coaxial breathing system over a period of at least 2 hours, preferably at least 12 or 24 hours, more preferably at least 3 days.

It is a further object of a preferred embodiment of the present invention to deliver more than 95%, preferably more than 98%, more preferably more than 99% of the absolute water vapour content of an inspiratory gas stream as output from a humidification chamber to a user proximal end of a coaxial breathing system.

The present invention provides a coaxial breathing system tubes having system user distal and proximal ends, the coaxial breathing system comprising an inlet tube for conveying inspiratory gases and an outlet tube for conveying expiratory gases, each of the inlet and outlet tubes having tube user distal and proximal ends, the tube user distal ends being in fluid communication with, in use, a fluid supply means and the tube user proximal ends being in fluid communication with, in use, a user, the inlet tube being coaxially arranged within the outlet tube; a temperature sensor, in use, at the system user proximal end; a heat source consisting essentially of a heater wire surrounded by an insulating hydrophobic layer and located in the outlet tube, the heat source being dimensioned and arranged to heat at least the expiratory gases in the outlet tube and the heat source, in use, being in electrical connection with a power supply means; and a regulating means for controlling actuation of the power supply means, the regulating means being in operative association with the temperature sensor.

Preferably, the regulating means provides that, in use, the inspiratory gases, at the user proximal end of the system, are heated to a predetermined temperature.

More preferably, the predetermined temperature, at the user proximal end of the system, is in the range of 37 - 40°C, optionally 37 - 39°C or 38 -39°C or 37.5 - 39.5°C.

Even more preferably, the predetermined temperature is measured in a common area intermediate the user proximal end of the system and, in use, a user.

It is generally held that, in due consideration of the water balance and heat balance of a patient's body, each inspiratory breath, as it enters the patient's lungs, is desired to have a temperature of not lower than 31°C. Thus, for example, US-A-4,463,755 suggests that the desired temperature range for the inhalant gas to be delivered to the patient's lungs is in the range of 32 - 35°C. The present invention, in a preferred embodiment, identifies that a somewhat higher, but narrow, temperature range of 37.5 - 40, preferably 38.5-39.5, °C, which is to be measured at the patient proximal end, most preferably in a common area, is desired. It will be appreciated that the upper limit of 40°C is determined by the normal upper limit of the temperature of the patient's lungs (38°C at maximum), plus 2°C to account for an expected 2°C fall in the temperature of the inspiratory breath during its passage from the present coaxial breathing system to the surface of the patient's lungs.

Preferably, the heat source is located, in addition, in the inlet tube, the heat source also being dimensioned and arranged to directly heat the inspiratory gases in the inlet tube.

In a preferred embodiment, the at least one heater wire is dimensioned to extend along at least 50%, preferably at least 70%, more preferably at least 90%, most preferably at least 95%, still more preferably, all of the length of either the outlet tube or of both of the inlet and the outlet tubes.

The heater wire is formed as a loop with both free ends electrically connected with an electrical connector. In a preferred embodiment, the loop is then fed in a rectilinear manner along the inlet tube towards the patient proximal end of the coaxial breathing system. The heater wire loop is then looped back, via the common area, through the outlet tube. In the outlet tube, the heater wire loop is preferably coiled. The free end of the heater wire loop is then fed through the T-piece into the return limb. Alternatively, the heat source is integral with either or both of the inlet and the outlet tubes.

The temperature sensor provides temperature data to the heater. If the temperature is less than the predetermined temperature, the heater is actuated and remains actuated until the temperature sensor records a temperature equal to, or greater than, the predetermined temperature, whereupon the heater is de-actuated.

This temperature regulation (on-off etc) of the heater continues while the coaxial breathing circuit of the present invention is in use.

The coaxial breathing systems of the present invention can be connected to fluid supply means such as respirators or ventilators, for use in respiratory care. Alternatively, the coaxial breathing systems of the present invention can be connected to fluid supply means for administering an anaesthetic and oxygen, for example, to patients, for use in anaesthetic circuits. In addition, the coaxial breathing systems of the present invention can be connected to fluid supply means for administering medicines, oxygen and the like, for use in inhalant therapy. All these coaxial breathing circuits share a basic construction which comprises an inlet circuit for connecting a fluid supply means to a user, via a tracheal catheter, or a face mask or the like fixated on the patient, and an outlet circuit for returned gas(es).

The user distal end of the inlet tube is adapted for substantially fluid tight communication either directly, or indirectly, with the fluid supply means. Inspiratory and expiratory valves are provided on the fluid supply means, in operative association, respectively, with the user distal ends of the inlet and outlet tubes. For example, the user distal end of the inlet tube may be in fluid-tight communication with an oxygen flow meter, via a pre-oxygenation /transportation adapter; an anaesthesia machine common gas outlet; an anaesthetic absorber unit; or an intensive care ventilator or the like. The user distal end of the outlet tube is adapted for substantially fluid-tight connection with either an anaesthetic absorber unit or an intensive care ventilator, in each case optionally, via a water trap limb for the collection of water condensate or, alternatively, to a Mapleson bagging limb or similar system for generating and/or maintaining air pressure and/or air volume for pulmonary hyperinflation.

Adjacent, in use, the user, the user proximal end of the inlet tube is adapted for substantially fluid-tight connection to, for example, an anaesthesia face mask, a tracheal tube or a laryngeal mask airway or like connection to the patient. Intermediate the user proximal ends of the inlet and outlet tubes and the patient, there is provided a common area in substantially fluid-tight connection with the anaesthesia face mask, the tracheal tube or the laryngeal mask airway or the like. In a preferred embodiment of the present invention, the temperature sensor is mounted in the common chamber, so that it is the gas temperature within the common area, which is measured. For example, for use with adults, a volume of 60-80ml for the common area is acceptable. The volume of the common area should be a fraction of the volume of an inspiratory breath. For example, the volume of the common area could be <80%, optionally <60%, alternatively <40%, further alternatively <30% or <20% of the volume of an inspiratory breath. This, of course, means that the volume of the common area needs to be modified depending on, for example, the body weight and general wellbeing of the patient. It will be apreciated that limiting the volume of the common area to a fraction of the volume of an inspiratory breath ensures that, in a most preferred embodiment, that the gas temperature is being measured in the so-called dead space of the present system where the gas temperature being measured reflects the temperature of the next breath to be inhaled, which is, in turn, a mixture of the last exhaled breath with fresh inspiratory gas(es). Without being bound by theory, it is believed that the temperature in this common area more accurately reflects the temperature of the next inspired breath and, thereby, optimises the temperature and humidity of each inspired (or inhaled) breath.

The coaxial breathing circuit of the present invention is intended for use in high- or low-flow gaseous anaesthesia, intensive care mechanical ventilation or continuous gas flow such as continuous positive airway pressure (CPAP) or bilevel positive airway pressure (BiPAP).

Minimising loss of active humidification is particularly important for those patients receiving long-term (i.e. more than 12 hours, for example more than 24 hours or even more than 48 or 72 hours) ventilation or the like.

The provision of a heat source in at least the outlet tube serves to minimise, for intensive care mechanical ventilation or continuous positive airway pressure, loss of active humidification, which has been provided by a humidification chamber such as a heated water bath. Thus, if the inlet tube of the coaxial breathing system accepts inspiratory gases from a humidification chamber at a temperature of 30-38°C, the coaxial breathing system of the present invention is suitable for heating the gas to the desired temperature, adjacent the user (preferably in the common area), in the range of 37.5-40°C, with a relative humidity (RH) in the range 100-85%, preferably 97.5-85%, more preferably 100-92%, most preferably 100-95% with an absolute humidity in the range 30-44, preferably 35-44, more preferably 41.8-44, most preferably, 40.5-44, mg H₂O/litre of gas in the next inspiratory (or inhaled) breath of the user.

Alternatively, it is envisaged that the coaxial breathing circuit of the present invention may be used for conveying heated inspiratory gases, which heated inspiratory gases have not been subjected to active humidification. Examples of situations where heated inspiratory gases are not necessarily subjected to active humidification include, but are not limited to, closed or semi-closed circuit anaesthesia and breathing systems for short-term, for example, less than 12, preferably less than 2, hours, use. Such a closed or semi-closed anaesthesia circuit would include an absorbent to absorb exhaled carbon dioxide. Such absorbents give off water vapour which raises the relative humidity above ambient humidity. Incorporation of a coaxial breathing circuit of the present invention into such a closed or semi-closed circuit anaesthesia system, which is solely heated by a heating means, but without active humidification, relative humidity and absolute humidity in the ranges aforementioned are maintained with or without the need for water traps for the collection of water condensate.

In a preferred embodiment, the heat source comprises at least one heater wire loop located within at least the outlet tube. The heater wire loop in the outlet tube may be arranged substantially rectilinearly, or, alternatively, in a spiral formation. Preferably, the heater wire loop is arranged in a spiral formation along the length of the outlet tube.

In a preferred embodiment, the heat source also comprises at least one heater wire loop located within the inlet tube. The heater wire loop may be arranged substantially rectilinearly, or, alternatively, in a spiral formation. Preferably, the heater wire loop in the inlet tube is arranged substantially rectilinearly along the length of the inlet tube.

In a preferred embodiment, the heat source also comprises a heater wire located within the inlet tube and then fed back through the outlet tube, thereby forming a loop.

It is preferred that the at least one heater wire loop has an outer diameter in the range of between 1-3.5mm with a watt density, within the portion or portions of the inlet and outlet tubes intended for heating, consistent with that required to deliver an inspiratory breath at the desired temperature and / or humidity and / or prevent formation of water condensate in the inlet tube and / or to limit or minimize formation of mobile water condensate in the outlet tube.

The total power density is, of course, limited by the power output of the voltage supply means. Usually, the total power density is intended to be in the range of between 10-100, preferably 20-70, more preferably 30-50, even more preferably 35-45, watts/metre of the coaxial breathing system, at an input current consistent with the specification of the voltage supply means, although it will be understood that the invention is not intended to be limited to these parameters. Hereinafter, where lengths of the coaxial breathing system are mentioned, it is intended that this be the sum of the respective lengths of the coaxial inlet and outlet tubes, divided by 2.

The length of the coaxial breathing system may be in the range of 1.0-2.5m but it is not intended to be so limited. Optionally, the length of the coaxial breathing system may be in the range of 1.25-2.0, preferably 1.5-1.8, m. If the maximum power output is about 60 W, the heat source in the form of at least one heater wire loop may have a total resistance, along its entire length of say 10 m, in the range of 4-15, for example 7-9, ohms and/or a power density in the range of 10-20 watts per metre of the sum of the coaxial breathing system, but, in each case, it is not intended to be so limited.

If a heat source is provided in both the outlet tube and the inlet tube, the power density of the heat source in the outlet tube may be in the range of 10-60, preferably 15-45, more preferably 20-40, still more preferably 25-35, W/m of the coaxial breathing system. In addition, the power density of the heat source in the inlet tube may be in the range of 2.5-25, preferably 5-15, more preferably 7.5-12.5, W/m of the coaxial breathing system.

The coaxial breathing system of the present invention is capable of operating with minute volumes of 1.51 to 301, but it is not intended to be so limited.

It will be appreciated that the incorporation of a heat source, within such a coaxial breathing system is of considerable advantage during intensive care respiratory therapy, where medium to long-term mechanical ventilation is required, by facilitating maintenance of the patient's natural heat and humidity balance, in particular, the patient's lung humidity.

It is within the scope of the present invention that the heat source, preferably the at least one heater wire, be adapted to have the same, or different, power densities per metre of tubing in each of the inlet and outlet tubes. In addition, it is within the scope of the present invention that the heat source, preferably the at least one heater wire, in either or both of the inlet or the outlet tube would have different power densities within different zones of each of inlet or the outlet tubes.

Alternatively or additionally, the heater wire may have a zoned resistance. For example, the heater wire may be present in the inlet tube but zoned (or modified) to have a relatively high resistance and, therefore, a negligible current. Further alternatively, the heater wire might be zoned (or modified) to achieve altered resistance and correspondingly altered currents, in different areas of either or both of the outlet and inlet tubes.

The invention will now be described with reference to the accompanying drawings, in which:

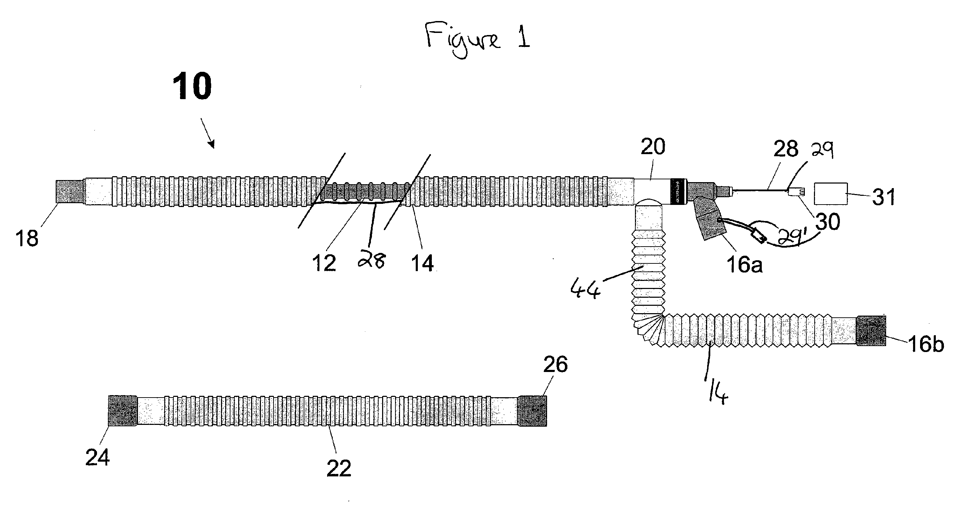

Figure 1 illustrates a side view of a coaxial breathing system according to the present invention;
Figure 2 illustrates an enlarged, partially sectioned view, of a portion of the coaxial breathing system of Figure 1;
Figure 3 illustrates a "patient simulation" rig to test the coaxial breathing system of the present invention;
Figure 4 illustrates an exploded perspective partial view of the patient proximal end of the coaxial breathing system of Figure 1;
Figure 5 illustrates a partial perspective cut-away view of an alternative coaxial breathing circuit of the present invention; and

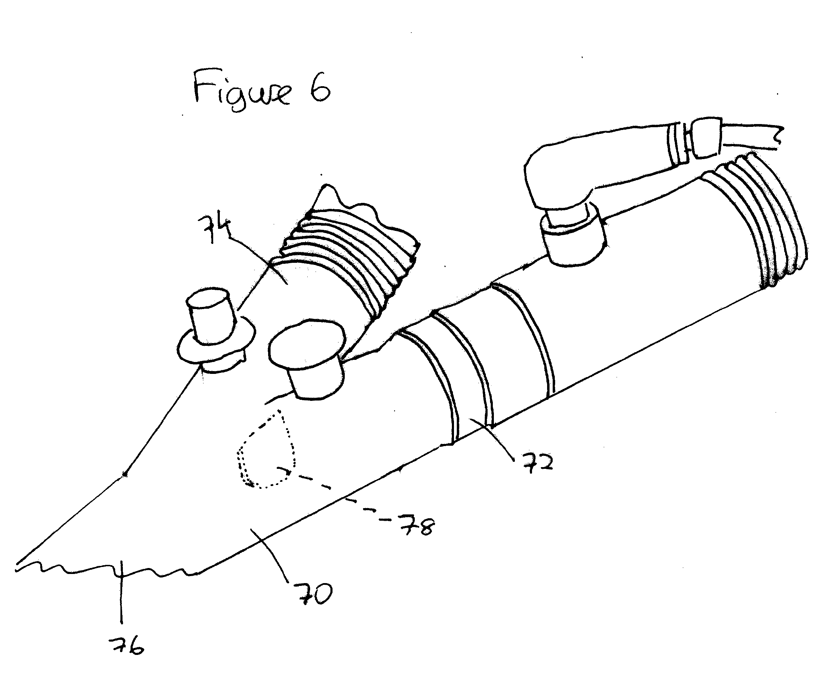

Figure 6 illustrates an exploded perspective partial view of the patient proximal end of a comparative non-coaxial breathing system, which is not of the present invention.

Referring now to the accompanying drawings, there is illustrated a breathing system, generally indicated as 10. The breathing system 10 comprises an inlet tube 12 located coaxially within an outlet tube 14. In the present embodiment, the inlet tube 12 may have an inner diameter of 15mm, whilst the outlet tube 14 may have an inner diameter of 26mm although the breathing system of the present invention is not intended to be limited to such dimensions.

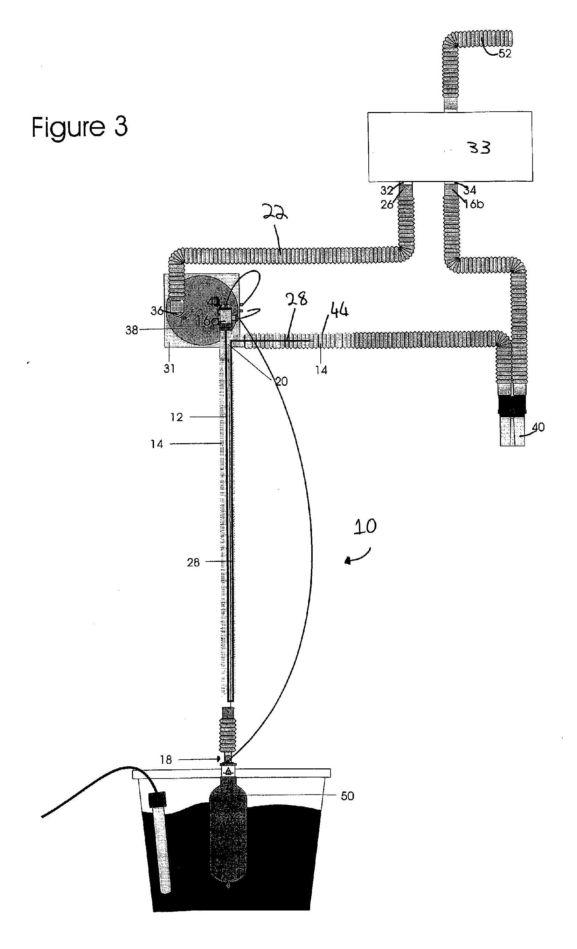

The breathing system 10 has user distal ends 16a and 16b distal a patient (not shown) and user proximal ends 18a and 18b proximal a patient. The user proximal ends 18a and 18b are adapted for connection with the patient in any conventional manner, whilst the ends 16a and 16b are adapted in the following manner. Specifically, the inlet user distal end 16a of the inlet tube 12 is connected to an outlet port end 41 of a humidification chamber 38. The inspiratory port 32 of the intensive care ventilator (shown as 33 in Figure 3) supplies fluid, for example air and anaesthetic, to the humidification chamber (shown as 38 in Figure 3), via a humidification limb 22, whose end 24 is connected to an inlet port 36 of the humidification chamber (shown as 38 in Figure 3), and whose end 26 is fluid-tightly connected to the inspiratory port 32 of a fluid supply means such as an anaesthesia machine or an intensive care ventilator or the like (shown as 33 in Figure 3). The outlet user distal end 16b of the outlet tube 14 is connected to an expiratory port (shown as 34 in Figure 3) of an anaesthetic absorber unit or an intensive care ventilator or the like (schematically shown as 33 in Figure 3), either directly or, optionally, via a return limb 44 and a water trap (shown as 40 in Figure 3).

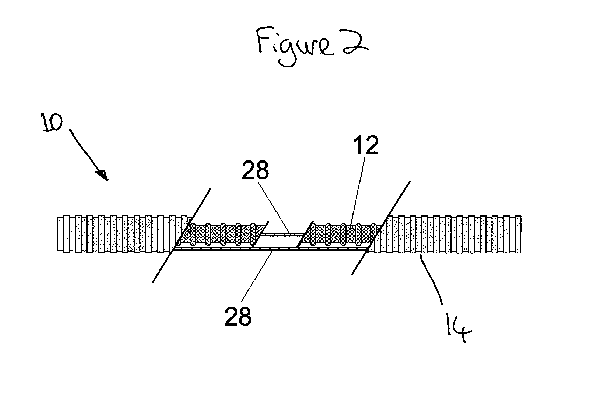

Referring in particular to Figure 2, a heater wire 28 is located along the length of the inlet tube 12 and is threaded in a continuous loop back along the length of the outlet tube 14, the respective free ends 29, 29' of the wire 28 being in operative electrical association, via a Y-piece electrical connector (shown schematically as 30) with a heating means in the form of a heater 31, of any conventional form. Alternatively, the present invention provides for the heater wire 28 to be located along the length of the outlet tube 14 and to be threaded in a continuous loop back along the length of the inlet tube 12, terminating at the electrical connector 30.

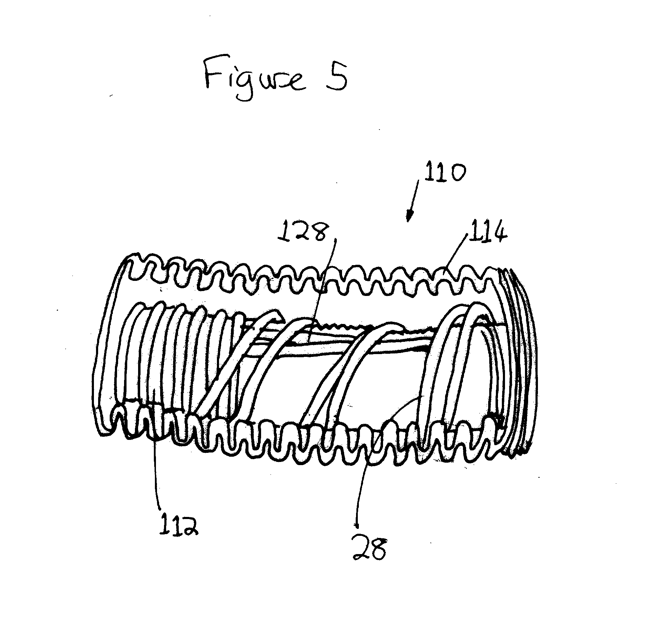

Further alternatively, the present invention provides for the heater wire 28 to be provided in the form of a loop whose free ends 29, 29' terminate, of course, at the electrical connector 30, the entire loop being located along the length of the outlet tube 14 and then threaded in a continuous loop back along the length of the inlet tube 12 (see Figure 5).

Even further alternatively, the present invention provides for the heater wire 28 to be provided in the form of a loop whose free ends 29, 29' terminate, of course, at the electrical connector 30, the entire loop being located along the length of the inlet tube 12 and to be threaded in a continuous loop back along the length of the outlet tube 14.

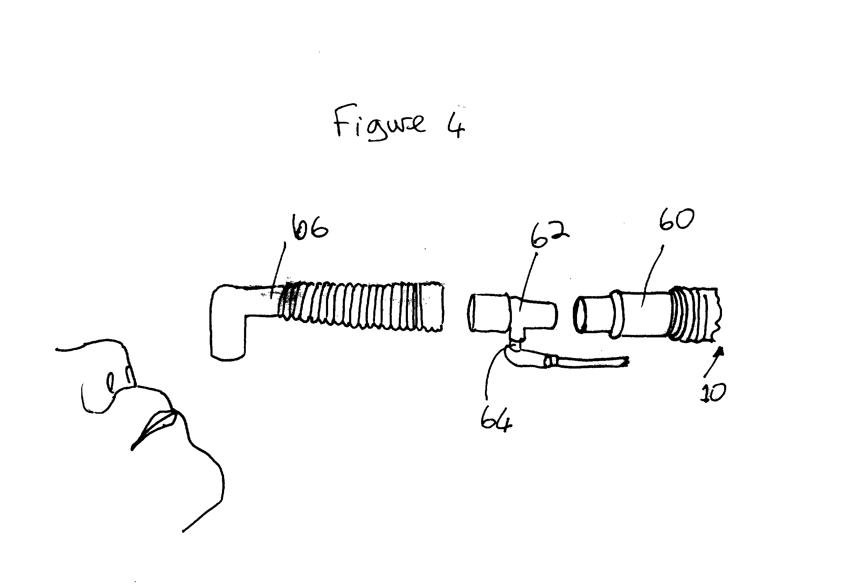

Referring to Figure 4, there is illustrated a partial exploded view, in perspective, of the user distal end of a breathing circuit 10 of the present invention. It will be observed that a common chamber 60 is fluid tightedly fixed to the outlet tube 14. The common chamber 60 is in fluid communication with both the inlet tube 12 and the outlet tube 14. Fluid tightedly fixed to the common chamber 60 is a hollow temperature measurement chamber 62, into which is fluid-tightedly mounted, a temperature sensor or probe 64. Finally, a mouth piece 66 or the like for connection with a patient is fluid-tightedly connected to the free end of the temperature measuring chamber 62.

It is an important feature of the present invention that the desired temperature measurement is carried out in one or more of the mouth piece 66, the temperature measuring chamber 62 and the common chamber 60. Without wishing to be bound by theory, the applicants believe that it is the desired temperature in the common area defined by these three chambers, which is important in determining the temperature of each inspiratory breath. It is, therefore, a feature of a preferred embodiment of the present invention that the temperature probe 64 be located in the common area as defined hereinabove.

In use, the inspiratory gases are conveyed in through the end 26 of the humidification limb 22, out the end 24 and, via a humidification chamber (shown as 38 in Figure 3) into the end 16a and, thereby, out the end 18a of the inlet tube 12, via the common area, into contact with a patient (not shown). The heater wire 28, being located along the length of the inlet tube 12, serves to heat the humidified inspiratory gases, and prevent water condensate within the inlet tube 12, before inhalation by the patient. The expired gases pass via the end 18b into the outlet tube 14 and via 20, out the end 16b and back to the gas supply means, in the form of an anaesthesia machine or a ventilator or the like (shown as 33 in Figure 3). In the present embodiment, the heater wire 28 is located along most of the length of the outlet tube 14, between ends 18b and 16b, and serves to heat the expiratory gases for the purposes of limiting or preventing the formation of mobile water condensate within the outlet tube 14.

In use, during an inhalation cycle by the patient, negative pressure is created within the coaxial breathing system, forcing an exhalation valve (not shown) closed. The simultaneous passage of inspiratory gases into the patient unseats an inhalation valve (not shown) so that the inspiratory gases can pass to the patient. During the exhalation cycle, positive pressure is exerted within the coaxial breathing system and the operation of the inhalation and exhalation valves is reversed.

### EXAMPLES

A coaxial breathing system 10 of the present invention, is used in conjunction with a heating means in the form of a heater humidifier, fitted with a humidification chamber. Inspiratory gases are passed through the humidification chamber, in order to saturate said gases with water vapour to achieve 95-100% RH and 39 - 37°C at the patient proximal end 18a of the coaxial breathing circuit 10 (taken as connection to an artificial lung, shown as 50 in Figure 3). The heater humidifiers and humidification chamber 38 are those manufactured by Fisher & Paykel (New Zealand).

A 'patient simulation' rig was developed, in order to test the breathing circuit 10 of the present invention with the said heater humidifiers and humidification chamber 38 in a way that simulated, in so far as is reasonably practicable, routine clinical practice with an adult intensive care patient (75kg bodyweight), undergoing standard mechanical ventilation. This rig is shown in Figure 3 of the accompanying drawings.

### Examples 1 - 5

The breathing circuit 10 of the invention was tested with the following two heater humidifiers, 730 series and 850 series and with a single design of humidification chamber - the MR290.

The present experimental setup comprised a ventilator (without an integral water trap); a 850 series heater humidifier; a MR290 humidification chamber 38 (water auto-fill with 135ml to fill level); a breathing circuit 10 of the present invention (with electrical connector 30 for the 850 series heater humidifier); and a condenser 52, which was fitted to the expiratory exhaust on the ventilator 30 to capture water from the expiratory flow, using a condensing temperature of 10°C.

The experimental parameters comprised an artificial lung 50 with a volume of 2.01 surrounded by water at constant 35-37°C. The experimental parameters also comprised a coaxial breathing circuit (inlet tube of length 1.6m, outlet tube of length 1.75m) fitted with heater wire.

The heater wire has a total length of 10m or a doubled or loop length of 5m. The free ends of the heater wire loop are electrically connected, via an electrical connector 30, to a 850 series heater humidifier. The heater wire loop is laid substantially rectilinearly along the complete 1.6m length of the inlet tube 12.
The balance of the 6.8m of the heater wire loop (doubled or looped length of 3.4m) is then looped back along the complete length of the outlet tube 14 in a loose spiral formation about the external surface of the inlet tube12; and the heater wire loop terminates in the return limb 44 of the outlet tube external of the coaxial breathing system of the present invention.

The heater wire as used herein has a total resistance of 7-9 ohms and a total wattage of 63-49 watts, using a 21 volt supply. If, of course, the resistance were lowered to say 4 ohms, a total power output of 110.25W could be achieved using the same 21 volt supply. Alternatively, if the potential supplied by the voltage supply means could be increased to say 30 volts, then the heater wire could still have a total resistance of 7-9 ohms but now with a total wattage of 128.5-100 watts. A man skilled in the art will appreciate that, to fulfill the objective of minimising occlusion of the inlet or outlet tubes with condensed water vapour, the outlet tube needs to be provided with heater wire having a power density sufficient to ensure that the temperature in the outlet tube drops by no more than 7°C, preferably no more than 5°C, during its passage along the outlet tube.

A breathing circuit inlet tube 12 was connected to a MR290 chamber 38, which, in turn, is further connected to a ventilator 33 inspiratory port 32, via 0.7m of standard corrugated tubing 22. A breathing circuit outlet tube 14 was connected to a ventilator 33 expiratory port 34 via a water trap 40 with a water-holding capacity of 65ml.

The operating parameters were 30-48% RH (ambient air); temperature of 18-21.2°C (ambient air); an inspiratory gas composition comprising ambient air at 30-48%% RH; a tidal volume of 500ml; minute volume 7,500ml (15 breaths per minute); total gas flow of 450l/hour; a total gas flow of 10,800l/24 hours; a peak flow velocity of 189ml/sec; and a test duration of 72 hours (Examples 1, 2, 3 and 4); and 2 hours (Example 5).

In each of Examples 1 and 2, the above-mentioned coaxial breathing circuit was fitted with heater wire loop as previously described, having a loop length of 1.6m, laid substantially rectilinearly along the complete length of the inlet tube 12 and then looped back along 1.75m of the length of the outlet tube 14 in spiral formation about the external surface of the inlet tube and terminating in the return limb 44 of the outlet tube 114. The heater wire (total length of 10m) has a total resistance of 7-9 ohms and a total wattage of 56-58 watts, using a 21 volt supply. This embodiment of the invention is illustrated in Figure 5, in which like parts are given similar numerals. Specifically, the wire loop 128 is fed along the inside of the inlet tube 112 and then looped along the inside of the outlet tube 114.

### Example 1:

The temperature of air leaving the humidification chamber was 35.5-37.5°C. This output air temperature was measured at the outlet port 16a before the start of the inlet tube 12. The saturation at this point was measured as 39mg H₂O vapour per litre of air (88.6% at 37°C).

The "patient" air temperature was measured at the end of the breathing circuit 10, before the artificial lung 50, at a mean of 39.2°C. The inlet tube 12 remained dry whilst at 95-100% RH at 39-37°C. The outlet tube 14 presented a fine water condensate that increased to a heavy 'bead' after 3 hours, then remained stable as a heavy 'bead' until termination at 72 hours. The artificial lung 50 collected 69.84g of water over 24 hours (2.91g/hr). The outlet tube 14 collected 5g water over 24 hours (0.21g/hr), substantially evenly distributed across the 1.75m length, with slightly heavier deposits towards the patient connection 18. This condensate was minimised in the outlet tube. The water trap 40 collected 190g over 24 hours (7.92g/hr). The condenser 52, on the ventilator 33 expiratory exhaust, collected 156 g water over 24 hours (6.5g/hr).

The relative humidity of the next inhaled breath was stable and constant at 95% at 39°C from 20 minutes into the test, measured in the common area. The relative humidity of the expiratory gas flow was 90-100% at 37°C from 20 minutes into the test, measured within the outlet tube 14, downstream of the common area.

The sum of the water condensed in the artificial lung 50, the outlet tube 14, the water trap 40 and the condenser 52 was 420.84g over 24 hours (17.53g/hr) or 39mg/l/air, which is 88.5% RH). This roughly corresponds with the measurement of water vapour output of the humidification chamber itself. The difference can be attributed to the limitation of the condenser at the exhaust port, which could not convert all of the expiratory flow to an anhydrous gas, simply by passing the gas through 10°C.

The total fluid output from the humidification chamber 38 was calculated from the sum of the condensed water in the artificial lung 50, in the outlet tube 14, in the water trap 40 and in the condensing limb 52. This was found to be 420.84g/24 hours (17,535mg/hr). When the total H₂O content is divided by the flow rate (l/hour), a figure of 38.96 mg H₂O vapour per litre of air at 37°C (88.5% RH) is derived. Thus, all of the water vapour output of the humidification chamber is detected in the common chamber adjacent the user proximal end of the coaxial system. This, in turn, shows that no water condensed in the inlet tube itself.

### Example 2

A limitation of the test rig of Example 1 was the lack of an allowance for absorption of water into the patient's bloodstream. If it is assumed that, in clinical use, the water collecting in the artificial lung would be less, then it can be assumed that the amount of condensate in the outlet tube 14 and water trap 40 would be decreased proportionally. Furthermore, it can be assumed that the potential for expiratory limb condensation to become mobile is decreased.

To test this hypothesis, 5g of a hygroscopic absorbent (anhydrous calcium chloride) was placed within the artificial lung 50. The results confirm the assumption that the outlet tube 14 will carry less condensation during clinical use, compared with our patient simulation rig.

This test shows that the water delivered in the next inhaled breath remained in vapour phase at 95% RH at 39°C until contact with the internal surface area of the artificial lung 50, where a portion of the transported water vapour was deposited in the artificial lung 50. The expiratory flow gases exited the artificial lung 50 at 90-100% RH at 39-37°C and remained largely in vapour phase, save for the cooling effect of the tubing surface causing minor condensate to form on the internal surface of this tubing. The majority of this flow remained in vapour phase until condensing in the water trap and after the exhaust port.

The results confirm the assumption that the outlet tube 14 will carry less condensation during clinical use, compared with our patient simulation rig.

### Example 3

### Description of wire lay-up:

A heater wire, of the resistance and power previously described, was assembled to the coaxial breathing system of the present invention, by passing the heater wire loop down the outlet tube in coiled formation around the outer surface of the inlet tube. The free ends 29, 29' of the heater wire to the outlet tube exit the coaxial breathing system of the present invention adjacent 20, through a port (not shown).

### Results

The inlet tube temperature in the common area reached 39°C at 90% RH without formation of water condensate at any part of the length of the inlet tube. A fine mist developed in the outlet tube, which did not accumulate to the same extent as Example 1. The results indicate that the transfer of heat from outlet tube to the gases in the inlet the inlet tube was sufficient to achieve the desired temperature of 39°C without allowing condensate to form along any part of the inlet tube.

### Comment

Using the total power of the heater wire within the outlet tube serves to maximise the prevention of mobile water condensate in the outlet tube, while simultaneously indirectly heating the gases in the inlet tube, by thermal transfer of heat through the material that forms the tubing wall of the inlet tube. It is accepted that the effectiveness of this assembly in clinical use, would be limited by the thickness of the said tubing wall. It would be desirable that it be as thin as possible and/or be manufactured from a material known to offer good thermal transfer properties or be of high thermal conductivity.

In clinical use, it is recognised that it is essential that no water condensate forms in the inlet tube, as this condensate could accumulate and become mobile water condensate, risking blockage of the inlet tube and its resultant effect in limiting or preventing adequate ventilation of the patient. In addition, the user may not become alerted to the problem as the invention may employ opaque tubing, preventing visualisation of condensate within the inlet tube. It is, therefore preferred, but not essential, to also provide a heat source in the inlet tube.

### Example 4

This embodiment of the coaxial breathing system of the present invention is primarily intended for use during closed circuit or low flow anaesthesiology using low fresh gas flows and employing recirculation of expiratory gases through a carbon dioxide absorbent, for the management of water condensate that accumulates in a conventional breathing circuit during this type [Y-piece and non-heated coaxial] of anaesthesia. The exothermic reaction during the absorption of carbon dioxide into the absorbent creates heat and humidity as the absorbent exchanges its moisture for carbon dioxide. This moisture is then deposited in the absorber canister and respiratory tubing, connected to the patient, as water condensate or vapourised water in the gas provided the gas temperature is high enough to hold the moisture as water vapour.

An embodiment of the invention for use in low flow anaesthesia is described:

The set up is the same as for Examples 1-3 without a humidification chamber and a heater humidifier but using a supply of 21v connected to the heater wire. The inlet port of a conventional absorber canister, with unidirectional valves complete with 2.0kg of carbon dioxide absorbent contained 16% water by weight, was connected to the outlet tube 16b. The outlet port of said absorber was connected to the inlet tube 16a. 250ml/min of carbon dioxide and 500ml/min of oxygen were introduced to the artificial lung, to simulate, respectively, the metabolic production of carbon dioxide during clinical closed circuit or low flow anaesthesia and the fresh gas flow of oxygen commonly used during clinical closed circuit or low flow anaesthesia. A ventilator was connected to the absorber canister to deliver the required tidal volume and breaths per minute. The exhaust valve on the absorber canister was set to vent to atmosphere at 45cmH₂O.

### Results

The inlet tube temperature reached 39°C without formation of water condensate at any part of the length of the inlet tube. The relative humidity of this inlet gas was <75% and >30%, preferably >50%. Water condensate did not form in the outlet tube and it can be presumed that the relative humidity here was <75% and >30%, preferably >50%.

### Comment

The heater wire was able to evaporate water condensate that attempted to form in the inlet and outlet tubes from the exothermic reaction in the absorber. This embodiment of the present invention may have benefits for avoiding the utilisation of water collection devices such as water traps during this anaesthetic technique. It is presumed that adequate humidity will be delivered to the patient via the associated use of a conventional passive heat and moisture exchanger positioned distal to and in fluid communication with the breathing system as close to the patient's airway or artificial airway as is possible.

### Comparative Example 5

The test rig comprised a ventilator (without integral water trap); a 850 Series heater humidifier; a MR290 humidification chamber 38 (water auto-fill with 135ml to fill level); a coaxial breathing circuit without the heat source as used in the present invention; and a condenser 52 was fitted to the expiratory exhaust 42 on the ventilator to capture water from the expiratory flow, using a condensing temperature of 10°C.

The test parameters comprise an artificial lung 50 volume 2.01 surrounded by water at constant 35-37°C; and a breathing circuit as described above. The breathing circuit inlet tube 12 is connected to the humidification chamber 38 via end 41, which is, in turn, further connected to ventilator 33 inspiratory port 32 via 0.7m of standard corrugated tubing. The breathing circuit outlet tube 14 is connected to ventilator 33 expiratory port 34 via a water trap 40 with a water-holding capacity of 65ml.

The test rig parameters were 30-48% RH (ambient air); temperature of 18-21.2°C (ambient air); an inspiratory gas composition comprising ambient air at 30-48% RH; a tidal volume of 500ml; a minute volume 7,500ml (15 breaths per minute); a flow of 450l/hour; a tidal volume of 500ml; a minute volume of 7,500ml (15 breaths per minute); a total flow of 10,800l/24 hours; a peak flow velocity of 189ml/sec; and a test duration of 2 hours.

### Results:

The test was terminated after 2 hours as mobile condensate accumulated in both the inlet (10ml) and outlet (15ml) tubes and completely occluded both of the gas pathways. This, in turn, caused the actuation of the safety mechanism in the ventilator. Accumulation of such volumes of water in the inlet and outlet tubes would require management of these tubes to periodically remove the water. In a clinical situation, this would lead, if no such human intervention occurred, to a failure to ventilate the patient. It is believed that this would not be acceptable clinical practice.

Water also accumulated in the artificial lung - 10ml within 2 hours. Water in the artificial lung reflects water that would be deposited in the patient lungs, risking asphyxiation. These results indicate that the system of the present comparative example would be inherently unsafe for clinical use.

### Comparative Example 6

### Y-piece ( or non-coaxial) breathing circuit with separate inspiratory and expiratory heater wires

This circuit supplied by Fisher & Paykel requires the humidifier to be set to achieve 39°C at the patient temperature probe, the temperature probe being located within the inlet tube. Figure 5 of the accompanying drawings illustrates an exploded perspective partial view of the patient proximal end of this comparative non-coaxial breathing system, which is not of the present invention. The common area herein comprises a Y-piece 70, having an inlet tube mouth 72, an outlet tube mouth 74 and a user proximal mouth 76. Within the Y-piece 70, there depends an internal partial septum 78, shaped and dimensioned to direct fresh inspiratory gas towards the user and to direct expiratory gas towards the outlet tube mouth and thence into the outlet tube. The temperature probe 64 is located upstream of this common area, within the inlet tube itself. The instructions for use for the Fisher & Paykel 730 series heater set the airway temperature to 39°C and the chamber control at -2°C. Following these instructions, gases are stated to leave the humidification chamber at 37°C and are heated by 2°C during their passage along the inlet tube, which results in 90% RH at the patient temperature probe 64. The distance between this point and the patient's teeth is intended to reduce the temperature by 2°C and hence provide a fully saturated inhaled gas mixture (100% RH). This distance can be as short as 50mm or as long as 200mm. Our tests show that, in practice, the inspiratory gases do reach 39°C at the patient temperature probe 64 but fail to drop 2°C over either of these distances so that the patient has a maximum humidity intake of 90% RH.

The humidification chamber is stated to have the ability to pick-up 44mgH₂O per 1 air at 37°C. Our tests show that only 37.5mg H₂O per 1 of air at 37°C enters into the inlet tube, reaching 39°C at the patient temperature probe within the inlet tube, failing then to reduce by 2°C, resulting in an inhaled gas of 35.88mg H₂O per 1 of air (80% RH at 39°C), thus under-humidifying the patient.

The invention is not limited to the embodiment described and illustrated herein which may be modified or amended without departing from the scope of the present invention.

## Claims

1. A coaxial breathing system tubes having system user distal and proximal ends, the coaxial breathing system comprising an inlet tube for conveying inspiratory gases and an outlet tube for conveying expiratory gases, each of the inlet and outlet tubes having tube user distal and proximal ends, the tube user distal ends being in fluid communication with, in use, a fluid supply means and the tube user proximal ends being in fluid communication with, in use, a user, the inlet tube being coaxially arranged within the outlet tube; a temperature sensor, in use, at the system user proximal end; a heat source consisting essentially of a heater wire surrounded by an insulating hydrophobic layer and located in the outlet tube, the heat source being dimensioned and arranged to heat at least the expiratory gases in the outlet tube and the heat source, in use, being in electrical connection with a power supply means; and a regulating means for controlling actuation of the power supply means, the regulating means being in operative association with the temperature sensor.

2. A breathing system according to Claim 1, in which the heat source is dimensioned and arranged to heat the expiratory gases in the outlet tube and, by heat exchange, the inspiratory gases in the inlet tube.

3. A breathing system according to Claim 1 or 2, in which the power density of the heat source in the outlet tube is in the range of 10-100, preferably 20-70, more preferably, 30-50, even more preferably 35-45,W/m of the coaxial breathing system.

4. A breathing system according to Claim 1, in which the heat source is additionally located in the inlet tube.

5. A breathing system according to Claim 4, in which the power density of the heat source in the outlet tube is in the range of 10-60, preferably 15-45, more preferably 20-40, still more preferably 25-35, W/m of the coaxial breathing system and the power density of the heat source in the inlet tube is in the range of 2.5-25, preferably 5-15, more preferably 7.5-12.5, W/m of the coaxial breathing system.

6. A breathing system according to Claim 1, in which the heat source is integral with the outlet tube.

7. A breathing system according to Claim 4, in which the heat source is integral with the inlet tube.

8. A breathing system according to any one of the preceding claims, in which the system user proximal end is in fluid communication with a common area, the common area being in fluid-tight communication with a patient, in use; the temperature sensor being arranged to measure the gas temperature within the common area.

9. A breathing system according to Claim 8, in which a gas temperature in the common area below a desired gas temperature actuates the regulating means, the regulating means, in turn, controlling actuation of the heat source, so that, in use, the desired gas temperature in the common area is attained.

10. A breathing system according to Claim 9, in which the desired temperature in the common area is in the range of 37.5-40, preferably, 38.5-39.5, °C.
